# EUROPEAN PATENT SPECIFICATION

(11) **EP 2 459 741 B1**
(45) Date of publication and mention of the grant of the patent: **01.01.2014**
(21) Application number: 10737900.0
(22) Date of filing: 28.07.2010
(51) Int. Cl.: C12Q 1/68

(54) **IN VITRO METHOD FOR PREDICTING WHETHER A COMPOUND IS GENOTOXIC IN VIVO**
In vitro Verfahren zur Vorhersage der Genotoxizität einer Substanz in vivo
Méthode in vitro pour la prédiction de la génotoxicité d'un composé in vivo

(30) Priority: 28.07.2009 EP 09166590
(43) Date of publication of application: 06.06.2012
(73) Proprietor: Universiteit Maastricht, 6211 LK Maastricht (NL); Rijksinstituut Voor Volksgezondheid En Milieu, 3721 MA Bilthoven (NL)
(72) Inventor: KLEINJANS, Joseph, Catharina, Stephanus, NL-6221 AR Maastricht (NL); VAN DELFT, Joseph, Henri, Marie, B-1040 ETTERBEEK (BE); MATHIJS, Karen, B-3990 Hamont (BE); PENNINGS, Jeroen, NL-3720 BA Bilthoven (NL); VAN KESTEREN, Petronella, Cornelia, Elisabeth, 3842 HK Harderwijk (NL); LUIJTEN, Mirjam, 3514 TP Utrecht (NL); VAN STEEG, Harmen, 1261 PG Blaricum (NL)
(74) Representative: Habets, Winand
(86) International application number: PCT/EP2010/060990
(87) International publication number: WO 2011/012665

(56) References cited:
- WO-A2-2006/050124
- MATHIJS KAREN ET AL: "Discrimination for genotoxic and nongenotoxic carcinogens by gene expression profiling in primary mouse hepatocytes improves with exposure time" TOXICOLOGICAL SCIENCES, ACADEMIC PRESS, SAN DIEGO, FL, US LNKD- DOI:10.1093/TOXSCI/KFP229, vol. 112, no. 2, 1 December 2009 (2009-12-01), pages 374-384, XP009126633 ISSN: 1096-6080 [retrieved on 2009-09-21]
- VAN DELFT J H M ET AL: "Comparison of supervised clustering methods to discriminate genotoxic from non-genotoxic carcinogens by gene expression profiling" MUTATION RESEARCH, ELSEVIER, AMSTERDAM, NL, vol. 575, no. 1-2, 4 August 2005 (2005-08-04), pages 17-33, XP004941724 ISSN: 0027-5107
- HU TING ET AL: "Identification of a gene expression profile that discriminates indirect-acting genotoxins from direct-acting genotoxins." MUTATION RESEARCH 18 MAY 2004, vol. 549, no. 1-2, 18 May 2004 (2004-05-18), pages 5-27, XP002559331 ISSN: 0027-5107
- UEHARA T ET AL: "A toxicogenomics approach for early assessment of potential non-genotoxic hepatocarcinogenicity of chemicals in rats" TOXICOLOGY, LIMERICK, IR, vol. 250, no. 1, 19 August 2008 (2008-08-19), pages 15-26, XP023315165 ISSN: 0300-483X [retrieved on 2008-05-29] cited in the application
- ELLINGER-ZIEGELBAUER H ET AL: "Comparison of the expression profiles induced by genotoxic and nongenotoxic carcinogens in rat liver" MUTATION RESEARCH, ELSEVIER, AMSTERDAM, NL, vol. 575, no. 1-2, 4 August 2005 (2005-08-04), pages 61-84, XP004941727 ISSN: 0027-5107 cited in the application
- ELLINGER-ZIEGELBAUER H ET AL: "Application of toxicogenomics to study mechanisms of genotoxicity and carcinogenicity" TOXICOLOGY LETTERS, ELSEVIER BIOMEDICAL PRESS, AMSTERDAM, NL, vol. 186, no. 1, 10 April 2009 (2009-04-10), pages 36-44, XP026001793 ISSN: 0378-4274 [retrieved on 2008-09-04] cited in the application
- MAGGIOLI J ET AL: "Toxicogenomic analysis methods for predictive toxicology" JOURNAL OF PHARMACOLOGICAL AND TOXICOLOGICAL METHODS, ELSEVIER, NEW YORK, NY, US, vol. 53, no. 1, 1 January 2006 (2006-01-01), pages 31-37, XP025015041 ISSN: 1056-8719 [retrieved on 2006-01-01]
- MATHIJS KAREN ET AL: "Assessing the metabolic competence of sandwich-cultured mouse primary hepatocytes" DRUG METABOLISM AND DISPOSITION, WILLIAMS AND WILKINS, BALTIMORE, MD, US, [Online] vol. 37, no. 6, 1 June 2009 (2009-06-01), pages 1305-1311, XP009126643 ISSN: 0090-9556

## Description

### Field of the invention

The invention is in the field of genomics and it provides an in vitro method for predicting whether a compound is genotoxic in vivo.

### Background of the invention

The classic 2 year rodent bioassay is the standard test for identifying the carcinogenic potential of chemical compounds. Such tests are time-consuming and costly. Moreover, they require the sacrifice of many animal lives. In vitro systems are therefore preferred; however, there is no reliable in vitro method for accurately predicting the genotoxicity of a compound in vivo.(1,2).

Well-established in vitro systems frequently used to identify the genotoxic potential of chemical compounds are for instance the bacterial Ames test, the mouse lymphoma assay, the micronucleus test and the chromosomal aberration test (3).

These classic in vitro genotoxicity tests, however, have been shown to generate an extremely high false positive rate when compared to in vivo carcinogenicity data. (3). False positive in this context means that the compound yields a positive result in the in vitro assay whereas it is negative for genotoxicity in an in vivo assay.

Because of the low predictive value of current in vitro assays, a compound that tested positive in an in vitro assay has to be retested in an in vivo assay in order to verify whether the compound is a true genotoxic (GTX) compound. This generates a lot of extra costs and efforts, as well as the sacrifice of many animal lives.

Therefore, new and more predictive in vitro systems are desired in the art which are capable of reliably discriminating genotoxins from non-genotoxins.

### Summary of the invention

The present invention employs the analysis of expression profiles of primary mouse hepatocytes as an in vitro system to discriminate GTX compounds from non-GTX compounds and also can predict whether a compound found positive in a conventional assay is a false GTX compound or a true GTX compound. It was found that differential expression of a number of genes could reliably predict whether a compound was a true genotoxic compound.

Hence, the invention relates to an in vitro method for distinguishing between genotoxic and non-genotoxic compounds by determining the expression level of at least genes 1700007K13Rik, GAS2L3, SPC25 and DDIT4L in primary mouse hepatocytes exposed to a potentially genotoxic compound and comparing the expression level thus obtained with a normal value of expression of said geneswherein it is concluded that a compound is genotoxic if the expression of at least one of said genes is increased at least two-fold.

### Detailed description of the invention

Chemical compounds, which are able to cause gene mutations or chromosomal damage in vivo are herein defined as true genotoxins (true GTX) (6, 12). False positive genotoxins (false positive GTX or false GTX) are herein defined as compounds that are not capable of causing gene mutations or chromosomal damage in vivo, but are positive in a conventional in vitro assay for genotoxicity.

Gene mutations or chromosomal damage may occur when the compound covalently binds to DNA in vivo. Such binding to DNA may be not or incorrectly repaired which may lead to mutations accumulating in time and ultimately inducing the formation of tumors (6, 12).

The present invention employs the analysis of expression profiles of primary mouse hepatocytes as an in vitro system to discriminate GTX from non-GTX compounds and also false GTX compounds from true GTX compounds. It was found that differential expression of a number of genes could reliably predict whether a compound was a true genotoxic compound. So as a first step in the method according to the invention, a culture of primary mouse hepatocytes is provided. The skilled person is aware of the various methods that may be used to obtain a culture of primary mouse hepatocytes. The examples provided herein may provide additional guidance.

An assay is described herein, capable of determining the expression of gene 1700007K13Rik. This gene is also known under its Genebank access code AK005731 or its Entrez Gene ID 69327. Assays that may determine gene expression are also known in the art. For instance, Mathijs et al., Toxicological Sciences, Academic Press, San Diego, FL, US LNKD-DOI:10.1093/TOXSCI/KFP229, vol 112, no2, 2009 pages 374 - 384 discloses a mouse hepatocyte based system for differentiating genotoxic and non-genotoxic compounds based on gene expression profiling.

Such an assay may consist of an assay capable of determining the expression of a single gene, such as a single PCR- based assay or a hybridization assay. In the alternative, a multiplex assay may be used, consisting of a plurality of different assays that can be performed simultaneously. This allows for the determination of simultaneous expression of more than one gene. Even more advantageously, the assay is a nucleic acid microarray such as a DNA microarray, such as a GeneChip® provided by Affymetrix.

Other genes that may be used in the determination of GTX from non-GTX compounds are listed in Tables 1 and 2. The genes provided in table 1 and 2 are readily accessible and identifiable for a person skilled in the art by their trivial name only. For reason of convenience, also the Genebank accession codes and Entrez Gene ID are given in table 1 and table 2. Primary sequences of these genes are published and can easily be retrieved from numerous public sources, such as Genebank.

**TABLE 1 Genes suitable in the method as described herein**

| **GENEBANK Access code** | **GENE SYMBOL** | **ENTREZ GENE ID** |
|---|---|---|
| | | |
| AK005731 | 1700007K13Rik | 69327 |
| AK010447 | Smyd3 | 69726 |
| BB318221 | Zdhhc14 | 224454 |
| BG261907 | Large | 16795 |
| Y15910 | Diap2 | 54004 |
| AV095209 | Mthfd1l | 270685 |
| AK019979 | 2610528E23Rik | 66497 |
| BC016073 | Cdkal1 | 68916 |
| BB821363 | Scfd2 | 212986 |
| AI596632 | Ptprg /// LOC632664 | 19270 |
| AW986246 | Maoa | 17161 |
| NM_028803 | Gbe1 | 74185 |
| AV141095 | 1110033M05Rik | 68675 |
| AF000969 | Cadps2 | 320405 |
| BB526605 | Mipol1 | 73490 |
| NM_008576 | Abcc1 | 17250 |
| BG070887 | Gtdc1 | 227835 |
| AW543460 | Pard3 | 93742 |
| BC016265 | Ube2e2 | 218793 |
| AV223474 | Zdhhc14 | 224454 |
| AI987929 | Ndrg1 | 17988 |
| AK009736 | Gpr137b /// LOC664862 /// LOC673335 | |
| AK007766 | 1810044A24Rik | 76510 |
| AK004419 | Fbxl17 | 50758 |
| AV173571 | 1700106N22Rik | 73582 |
| BB308836 | Ppm1l | 242083 |
| BC004827 | Psat1 | 107272 |
| AW240761 | Tbc1d5 | 72238 |
| BG066903 | Kif16b | 16558 |
| NM_025770 | Atg10 | 66795 |
| BC025915 | Cova1 | 209224 |
| NM_018770 | Igsf4a | 54725 |
| AF022072 | Grb10 | 14783 |
| BC025837 | Sbk1 | 104175 |
| BG076151 | Ppm1d | 53892 |
| BF719766 | Thyn1 | 77862 |
| AV377066 | 9130221J18Rik | 102123 |
| BG065754 | Ccng1 | 12450 |
| BC025501 | Aaas | 223921 |
| NM_134188 | Acot2 | 171210 |
| NM_021451 | Pmaip1 | 58801 |
| BC026422 | Tgm1 | 21816 |
| BC015270 | Hist2h3c2 | 97114 |
| NM_053168 | Trim11 | 94091 |
| BB027848 | 4732466D17Rik | 212933 |
| AV327248 | Zfp365 /// LOC674611 | |
| AV219418 | Ldhb | 16832 |
| BG069873 | Gnb1l | 13972 |
| AF204959 | Cyp3a25 /// LOC622249 | 56388 |
| NM_030697 | Ankrd47 | 80880 |
| BM198879 | Ercc5 | 22592 |
| AW543723 | | |
| AK014608 | 4632434I11Rik | 74041 |
| AV298304 | Homez | 239099 |
| BC012260 | Psmf1 | 228769 |
| NM_013866 | Zfp385 | 29813 |
| AF065917 | Lif | 16878 |
| AF297615 | Ggta1 | 14594 |
| BB770528 | Rai2 | 24004 |
| BC012247 | Dcxr | 67880 |
| NM_011316 | Saa4 | 20211 |
| NM_007987 | Fas | 14102 |
| BI660702 | EII3 | 269344 |
| BM230508 | A030007D23Rik | 319530 |
| AI594683 | Dmn | 233335 |
| NM_011176 | St14 | 19143 |
| BB463610 | 4632434I11Rik | 74041 |
| BC019882 | Acaa1b | 235674 |
| AK007854 | 1810053B23Rik | 69857 |
| BC010462 | BC010462 | 209588 |
| BB043558 | 9230114K14Rik | 414108 |
| NM_008522 | Ltf | 17002 |
| NM_012006 | Acot1 | 26897 |
| BB275142 | AW456874 | 218232 |
| BC008626 | Icam1 | 15894 |
| BI651416 | Cdc42bpg | 240505 |

**TABLE 2 Genes suitable in the method as described herein**

| **GENEBANK Access code** | **GENE SYMBOL** | **ENTREZ GENE ID** |
|---|---|---|
| AK005731 | 1700007K13Rik | 69327 |
| BI651416 | Cdc42bpg | 240505 |
| NM_008522 | Ltf | 17002 |
| BB043558 | 9230114K14Rik | 414108 |
| NM_007987 | Fas | 14102 |
| BC022148 | Ces5 | 234673 |
| BC019882 | Acaa1b | 235674 |
| BB463610 | 4632434I11Rik | 74041 |
| BM230508 | A030007D23Rik | 319530 |
| AI594683 | Dmn | 233335 |
| AV327248 | Zfp365 /// LOC674611 | |
| BE956581 | Cpt1c | 78070 |
| NM_011176 | St14 | 19143 |
| BM200015 | Hsdl2 | 72479 |
| BB223872 | Bscl2 | 14705 |
| AF297615 | Ggta1 | 14594 |
| BC027026 | Cdkn2c | 12580 |
| NM_012006 | Acot1 | 26897 |
| AK014608 | 4632434I11Rik | 74041 |
| BC012247 | Dcxr | 67880 |
| BC027121 | Spbc25 | 66442 |
| BG797099 | Ddit4l | 73284 |
| BB743970 | BC015286 | 234669 |
| BF719766 | Thyn1 | 77862 |
| BC027185 | 2210023G05Rik | 72361 |
| AF033112 | Siva | 30954 |
| BG065754 | Ccng1 | 12450 |
| BB781615 | 6530418L21 Rik | 109050 |
| BC013893 | Masp2 | 17175 |
| BC003284 | Wdr21 | 73828 |
| BC006713 | Dgka | 13139 |
| NM_011075 | Abcb1b | 18669 |
| BB009155 | | |
| BG967046 | Tbc1d2 | 381605 |
| NM_030697 | Ankrd47 | 80880 |
| BB275142 | AW456874 | 218232 |
| AV246296 | Eda2r | 245527 |
| NM_013738 | Plek2 | 27260 |
| NM_018881 | Fmo2 | 55990 |
| BM936480 | Fmo2 | 55990 |
| BM198879 | Ercc5 | 22592 |
| AK018383 | Tmem19 | 67226 |
| AV254764 | | |
| BC021352 | Plod2 | 26432 |
| BB027848 | 4732466D17Rik | 212933 |
| AK017734 | Tmem14a | 75712 |
| AF069954 | Bscl2 | 14705 |
| BB770528 | Rai2 | 24004 |
| NM_009897 | Ckmt1 | 12716 |
| AK007854 | 1810053B23Rik | 69857 |
| B1966443 | Itm2a | 16431 |
| NM_013929 | Siva | 30954 |
| BG076151 | Ppm1d | 53892 |
| AV251625 | Ddit4l | 73284 |
| AV219418 | Ldhb | 16832 |
| NM_011316 | Saa4 | 20211 |
| NM_007980 | Fabp2 | 14079 |
| BB046347 | Mycbp | 56309 |
| AF335325 | Ddit4l | 73284 |
| AK010738 | Ascl2 | 17173 |
| NM_134188 | Acot2 | 171210 |
| NM_008935 | Prom 1 | 19126 |
| BB140436 | Slc16a10 | 72472 |
| NM_019738 | Nupr1 | 56312 |
| X62701 | Plaur | 18793 |
| AV141095 | 1110033M05Rik | 68675 |
| AI747296 | Gmds | 218138 |
| BC005552 | Asns | 27053 |
| BB458460 | Chchd6 | 66098 |
| BG076333 | Mthfd2 | 17768 |
| AK019979 | 2610528E23Rik | 66497 |
| AV095209 | Mthfd1l | 270685 |
| AV216768 | Phgdh /// LOC668771 /// LOC671972 /// LOC673015 | |
| AV221299 | Gfra1 | 14585 |
| BQ174991 | Chsy1 | 269941 |
| NM_013642 | Dusp1 | 19252 |
| L21027 | Phgdh /// LOC666422 /// LOC666875 /// LOC669985 /// LOC671102 /// LOC673015 /// LOC675010 | 236539 |
| BB204486 | Phgdh /// LOC382931 /// LOC384524 /// LOC385344 /// LOC547171 /// LOC627427 /// LOC666422 /// LOC6 | |
| BC025169 | Chac1 | 69065 |
| BC026131 | Slc7a5 | 20539 |
| BC010318 | Pck2 | 74551 |
| BB730977 | Cachd1 | 320508 |
| AA561726 | Phgdh /// LOC668771 /// LOC670155 /// LOC671972 /// LOC673015 | |
| BC012955 | Trib3 | 228775 |
| BC004827 | Psat1 | 107272 |
| NM_007556 | Bmp6 | 12161 |
| NM_134147 | D930010J01Rik | 107227 |
| AV173869 | D14Ertd171e | 238988 |
| AF022072 | Grb10 | 14783 |
| BC019379 | Gprk5 | 14773 |
| AK010447 | Smyd3 | 69726 |
| BC017615 | Slc24a3 | 94249 |
| BB246912 | 1700112E06Rik | 76633 |
| AF000969 | Cadps2 | 320405 |
| BG066491 | Fhod3 | 225288 |
| AF055573 | Fhit | 14198 |
| NM_053122 | Immp2l | 93757 |

In another step of a method as disclosed herein, the compound to be tested is contacted with the primary mouse hepatocytes. The skilled person will be aware of the metes and bounds of this step. In the examples section, the concentrations used for 10 true and false GTX compounds are provided as guidance. In general, the use of cytotoxic concentrations should be avoided. The skilled person will know how to avoid using cytotoxic concentrations of test compounds.

It was found to be useful to measure the gene expression in primary mouse hepatocytes at two consecutive moments in time or at two different intervals. These moments should be chosen empirically depending on a suitable expression pattern of the gene 1700007K13Rik or the genes listed in table 1 and 2 in the particular primary mouse hepatocytes chosen for the method. In general however, intervals of 1 to 2 days were found most appropriate. In the particular examples shown, it was chosen to analyse the gene expression at 24 hours and 48 hours after contacting the mouse hepatocytes with the test compound. This was found to produce very satisfying results.

In order to obtain reproducible results, it was found advantageous to obtain at least three independent readings of the gene expression. Hence, the above steps may be repeated at least twice to obtain a more reproducible and reliable result.

The results of the gene expression analysis may then be fed into a computer program capable of performing a supervised classification analysis. This method was found to provide superior results as compared to unsupervised classification methods and hierarchical clustering methods.

Supervised learning methods are computational approaches for class prediction based on biological data, such as generated with microarrays. Several methods have been shown to perform well with microarray data. Examples are support vector machines (SVM), RandomForest (RF), k-nearest neighbours (KNN), diagonal linear discriminant analysis (DLDA), shrunken centroids (PAM), classification and regression trees (CART), probabilistic neural network (PNN) and Weighted Voting (WV). The shrunken centroids software (Prediction Analysis of Microarray, PAM, Version 2.1 (Sep 14, 2005), http://www-stat.stanford.edu/∼tibs/PAM/; Tibshirani et al. PNAS 2002 99:6567-6572) was used as described in the examples for identifying genes.

Such computer programs may be trained with a data set obtained for known true GTX and false GTX compounds at the intervals chosen, such as presented in Tables 5-8. These programs, when trained with a suitable data set, can be used to predict whether a compound is a GTX compound or a non-GTX compound or for distinguishing false GTX from true GTX. This is based on a computational comparison of expression of said at least one gene with and without the test compound at the two consecutive moment in time with data from reference compounds (e.g. provided in Table 5-8) by means of a supervised classification method.

By repeating the steps of treating cells with the compound at least 2 times to obtain at least three measurements, at least six independent preliminary predictions can be obtained for the genotoxicity of a test compound; 3 repeats at two consecutive moments in time. These data may then be converted into a final prediction for the genotoxicity of a test compound by using the algorithm provided in table 3.

**Table 3**

| **Prediction at first time point** | **Prediction at second time point** | **Final prediction** |
|---|---|---|
| 3 repeats False positive | 3 repeats False positive | False-positive genotoxic = Not Genotoxic *in vivo* |
| 3 repeats False positive | 2 repeats False positive | False-positive genotoxic = Not Genotoxic *in vivo* |
| 3 repeats False positive | 1 repeats False positive | Equivocal = no prediction possible yet |
| 3 repeats False positive | 0 repeats False positive | True genotoxic = Genotoxic *in vivo* |
| 2 repeats False positive | 3 repeats False positive | False-positive genotoxic = Not Genotoxic *in vivo* |
| 2 repeats False positive | 2 repeats False positive | False-positive genotoxic = Not Genotoxic *in vivo* |
| 2 repeats False positive | 1 repeats False positive | Equivocal = no prediction possible yet |
| 2 repeats False positive | 0 repeats False positive | True genotoxic = Genotoxic *in vivo* |
| 1 repeats False positive | 3 repeats False positive | Equivocal = no prediction possible yet |
| 1 repeats False positive | 2 repeats False positive | Equivocal = no prediction possible yet |
| 1 repeats False positive | 1 repeats False positive | True genotoxic = Genotoxic *in vivo* |
| 1 repeats False positive | 0 repeats False positive | True genotoxic = Genotoxic *in vivo* |
| 0 repeats False positive | 3 repeats False positive | True genotoxic = Genotoxic *in vivo* |
| 0 repeats False positive | 2 repeats False positive | True genotoxic = Genotoxic *in vivo* |
| 0 repeats False positive | 1 repeats False positive | True genotoxic = Genotoxic *in vivo* |
| 0 repeats False positive | 0 repeats False positive | True genotoxic = Genotoxic *in vivo* |

Hence, described herein is a method for distinguishing between genotoxic and non-genotoxic compounds by determining the expression level of at least gene 1700007K13Rik in primary mouse hepatocytes exposed to a potentially genotoxic compound and comparing the expression level thus obtained with a normal value of expression of said at least gene 1700007K13Rik wherein it is concluded that a compound is genotoxic if the expression of said at least gene 1700007K13Rik is increased at least two-fold.

The method as described herein may even be improved by analyzing more than one gene. Preferably the method employs the detection of the expression level of at least one additional gene selected from the group consisting of gene GAS2L3 (237436), gene SPC25 (66442) and gene DDIT4L (73284). Also the method may be improved by adding at least one additional gene selected from the group consisting of the genes provided in table 1 and table 2, such as 2 genes or more than 2, such as 3, 4, 5, 6, 7, 8, 9, 10, 11, 12, 13, 14, 15, 16, 17, 18, 19, 20, 21, 22, 23, 24, 25, 26, 27, 28, 29 or even 30 or more genes selected from the genes provided in table 1 and table 2.

In a method as described herein, at least 3 measurements of expression of said at least one gene with and without the test compound at the two consecutive moments in time are compared with data obtained from known true genotoxic compounds. The true GTX compounds used in the study as presented here are known genotoxic compounds as well as known carcinogens. They exhibit a positive result in several in vitro assays as well as several in vivo models (Table 4). They are known to induce genotoxicity in vivo.

The false GTX compounds used in the present study are known non-genotoxic compounds in vivo as well as non-carcinogens in vivo. They only show a (false) positive result in certain in vitro tests, but are known not to induce genotoxicity in vivo. These compounds are also listed in table 4.

The non-genotoxic carcinogens used in the study (Table 4) are known carcinogens in vivo, but do not induce genotoxicity in vivo, nor with in vitro tests. The non-carcinogens used in the study are not known as carcinogens and do not cause genotoxicity in vivo, nor with in vitro tests.

**Table 4. Overview of the compounds used in primary mouse hepatocyte exposure**

| **Chemical** | **Abbreviation** | **CAS nr.** | **Concentration** | **Vehicle** |
|---|---|---|---|---|
| | | | | |

| *True GTX compounds* | | | | |
|---|---|---|---|---|
| Benzo(a)pyrene | BaP | 50-32-8 | 30 µM | DMSO |
| Aflatoxin B1 | AFB1 | 1162-65-8 | 10 µM | DMSO |
| 2-Acetylaminofluorene | 2-AAF | 53-96-3 | 125 µM | DMSO |
| Dimethylnitrosamine | DMN | 62-75-9 | 5 µM | PBS |
| Mitomycin C | MitC | 50-07-7 | 5 µM | PBS |
| Para-Cresidine | pCres | 120-71-8 | 8mM | DMSO |
| | | | | |

| *False GTX compounds* | | | | |
|---|---|---|---|---|
| o-Anthranilic acid | ANAC | 118-92-3 | 2 mM | DMSO |
| 2-(Chloromethyl)pyridine.HCl | 2-CP | 6959-47-3 | 125 µM | DMSO |
| 4-Nitro-o-phenylenediamine | 4-NP | 99-56-9 | 2mM | DMSO |
| Quercetin | Q | 117-39-5 | 200 µM | DMSO |
| 8-Hydroxyquinoline | 8-HQ | 148-24-3 | 150 µM | Ethanol |
| | | | | |

| *Non-genotoxic carcinogens* | | | | |
|---|---|---|---|---|
| Cyclosporin A | CsA | 59865-13-3 | 10 µM | DMSO |
| 2,3,7,8-Tetrachlorodibenzodioxin | TCDD | 1746-01-6 | 200 nM | DMSO |
| Carbon tetrachloride | CCl4 | 56-23-5 | 1 mM | DMSO |
| Tetradecanoylphorbol Acetate | TPA | 16561-29-8 | 1 µM | DMSO |
| Wy-14,643 | Wyeth | 50892-23-4 | 300 µM | DMSO |
| | | | | |

| *Non-genotoxic Non-carcinogens* | | | | |
|---|---|---|---|---|
| Bisphenol A | BPA | 80-05-7 | 100 µM | DMSO |
| Diclofenac | DF | 15307-86-5 | 300 µM | DMSO |
| Bis(tri-n-butyltin)oxide | TBTO | 56-35-9 | 300 nM | Ethanol |
| Amiodarone | AMD | 1951-25-3 | 10 µM | DMSO |
| D-Mannitol | dMan | 69-65-8 | 2 mM | DMSO |

Table 4a provides the log2-gene expression ratios.

**Table 4a: Ratios of gene expression treated/untreated**

| | **1700007K13RIK** | **GAS2L3** | **SPC25** | **DDIT4L** |
|---|---|---|---|---|
| *genotoxic carcinogens* | | | | |
| BaP | 2,46394 | 1,19836 | 1,28371 | 2,06696 |
| AFB | 2,91154 | 1,23443 | 1,52318 | 1,61622 |
| DMN | 2,14075 | 0,21656 | 0,83678 | 0,76622 |
| MMC | 4,95769 | 1,55118 | 2,1958 | 2,7547 |
| pCres | 0,20861 | 1,56162 | 1,33141 | 1,06569 |

| *Nongenotoxic carcinogens* | | | | |
|---|---|---|---|---|
| CsA | -0,0019 | -0,4304 | 0,19765 | 0,64756 |
| TCDD | 0,0752 | 0,21999 | 0,01802 | 0,26458 |
| CCl4 | 0,39061 | -0,3188 | 0,35438 | 0,48935 |
| TPA | -0,0617 | 0,01747 | 0,03809 | 0,05594 |
| Wyeth | -0,3527 | -0,7113 | -0,0756 | -0,1013 |

| *Noncarcinogens* | | | | |
|---|---|---|---|---|
| BPA | -0,0382 | -0,06 | -0,0457 | 0,0293 |
| DF | 0,09622 | -0,2839 | 0,40792 | 0,12681 |
| TBTO | 0,09131 | -0,0161 | 0,21862 | 0,31897 |
| AMD | -0,2612 | 0,27578 | -0,4118 | -0,309 |
| dMan | 0,02382 | -0,2175 | -0,0363 | 0,03421 |

Hepatocyte cells were incubated and exposed to a compound for 24 h before being harvested for RNA isolation. In order to get reproducible data, four independent replicate biological experiments with compound-exposed hepatocytes from different mice were conducted for each compound and gene expression data were compared to four independent replicate biological experiments with control- or vehicle-exposed hepatocytes from different mice.

The results of the gene expression analysis may then be fed into a statistical software package such as R, Splus, or Microsoft Excel. For genes that are able to discriminate between GTX and non-GTX compounds, differential expression can be scored on a gene-by-gene basis. We found it advantageous to use the following scoring system: A point was scored if two criteria were met, (a) if the gene expression values for the four compound-exposed samples differed significantly from the vehicle-exposed samples with a t-test p-value < 0.01; (b) if the average gene expression value for the four compound-exposed samples was at least twice that of the average vehicle-exposed samples. If none or only one of these criteria were met, no point was scored.

After the discriminating genes are scored, the statistical software can be used to compare the total number of positive genes between genotoxic and non-genotoxic compounds and apply a suitable threshold to discriminate between classes, Using said four genes mentioned in the table (1700007K13RIK, GAS2L3, SPC25, DDIT4L), we found that if this score is 0, a compound can be believed not to be a genotoxic carcinogen. If this score is 1, 2, 3 or 4, a compound is genotoxic.

For each of the four genes mentioned in the table (1700007K13RIK, GAS2L3, SPC25, DDIT4L), a statistical comparison was made between the normalized expression data for these genes. A point was scored if two criteria were met, (a) if the gene expression values for the four compound-exposed samples differed significantly from the vehicle-exposed samples with a t-test p-value < 0.01; (b) if the average gene expression value for the four compound-exposed samples was at least twice that of the average vehicle-exposed samples. If none or only one of these criteria were met, no point was scored.

For each compound, the sum of the scores for four genes is taken. If this score is 0, a compound can be believed not to be genotoxic. If this score is 1, 2, 3 or 4, a compound is genotoxic.

**Table 4b; scores for each of the four genes**

| | **1700007K13RIK** | **GAS2L3** | **SPC25** | **DDIT4L** | **Total score** |
|---|---|---|---|---|---|
| BaP average | 1 | 1 | 1 | 1 | 4 |
| AFB average | 1 | 1 | 1 | 1 | 4 |
| DMN average | 1 | 0 | 0 | 0 | 1 |
| MMC average | 1 | 1 | 1 | 1 | 4 |
| pCres average | 0 | 1 | 1 | 1 | 3 |
| CsA average | 0 | 0 | 0 | 0 | 0 |
| TCDD average | 0 | 0 | 0 | 0 | 0 |
| CCl4 average | 0 | 0 | 0 | 0 | 0 |
| TPA average | 0 | 0 | 0 | 0 | 0 |
| Wyeth average | 0 | 0 | 0 | 0 | 0 |
| BPA average | 0 | 0 | 0 | 0 | 0 |
| DF average | 0 | 0 | 0 | 0 | 0 |
| TBTO average | 0 | 0 | 0 | 0 | 0 |
| AMD average | 0 | 0 | 0 | 0 | 0 |
| dMan average | 0 | 0 | 0 | 0 | 0 |

For the combination of the particular mouse hepatocytes and time intervals chosen in the study exemplified in the examples, the data obtained with the true GTX compounds at 24 and 48 hours are provided in table 5 and table 6 respectively. The corresponding data obtained with the false GTX compounds is provided in table 7 and table 8 respectively.

**Table 5 Gene expression data at 24 hr with true GTX compounds.**

| **GENEBANK ACCESS CODE** | **AFB1** | **BaP** | **2-AAF** | **DMN** | **MitC** |
|---|---|---|---|---|---|
| | **24 h** | **24 h** | **24 h** | **24 h** | **24 h** |
| | **average** | **average** | **average** | **average** | **average** |
| AK010447 | -1,2553 | -1,0413 | -0,17513 | -1,0358 | -1,43853 |
| BB318221 | -1,0808 | -1,67917 | -0,41097 | -1,04093 | -2,70633 |
| BG261907 | -1,11937 | -1,3429 | -0,25017 | -1,3871 | -3,87647 |
| Y15910 | -1,88907 | -1,35203 | -0,36603 | -1,63633 | -2,81277 |
| AV095209 | -0,83977 | -0,58853 | 0,4403 | -1,00697 | -2,45413 |
| AK019979 | -2,53743 | -0,6154 | 0,324767 | -1,32373 | -3,31723 |
| BC016073 | -1,09967 | -0,6631 | -0,1464 | -1,24277 | -1,96257 |
| BB821363 | -1,35393 | -1,40327 | -0,1216 | -1,143 | -2,48487 |
| AI596632 | -1,9945 | -1,8362 | -0,0981 | -1,90023 | -4,79467 |
| AW986246 | -0,33847 | 0,1617 | 0,018533 | -0,37203 | -0,21193 |
| NM_028803 | -1,00427 | -0,54163 | -0,2716 | -1,10333 | -1,8898 |
| AV141095 | -1,29513 | -1,05753 | -0,28807 | -1,37477 | -2,28737 |
| AF000969 | -2,16997 | -1,90953 | -0,53713 | -1,03907 | -2,40647 |
| BB526605 | -0,7181 | -0,7419 | 0,346567 | -1,5816 | -2,03513 |
| NM_008576 | -0,18853 | 0,1024 | 0,332067 | -0,92953 | -1,31177 |
| BG070887 | -1,47347 | -0,97337 | -0,01607 | -1,11183 | -2,35577 |
| AW543460 | -0,2756 | -0,61937 | -0,3156 | -1,4802 | -3,16427 |
| BC016265 | -0,87973 | -0,75103 | -0,39843 | -1,28743 | -2,20177 |
| AV223474 | -1,02503 | -1,37517 | -0,44587 | -0,9067 | -2,28283 |
| AI987929 | -0,8074 | -0,36477 | 0,8432 | -0,98383 | -3,32443 |
| AK009736 | -0,50757 | 0,217433 | -0,00723 | -0,83673 | -1,05647 |
| AK007766 | -1,55373 | -1,28833 | 0,010033 | -0,8706 | -2,22737 |
| AK004419 | -1,36467 | -1,00123 | -0,16797 | -1,22027 | -4,0931 |
| AV173571 | -1,239.13 | -1,12323 | -0,04133 | -1,0106 | -2,28717 |
| BB308836 | -1,07683 | -0,61223 | -0,14993 | -0,98813 | -1,64007 |
| BC004827 | -1,0032 | -0,04097 | 0,279733 | -1,51817 | -2,4733 |
| AW240761 | -1,11773 | -0,703 | -0,0924 | -1,19117 | -2,64317 |
| BG066903 | -0,50743 | -0,13297 | -0,21247 | -0,86817 | -1,04667 |
| NM_025770 | -1,12757 | -1,05323 | 0,016633 | -0,51127 | -1,32903 |
| BC025915 | -0,64197 | -0,53963 | -0,15683 | -0,95017 | -1,61897 |
| NM_018770 | -0,43897 | -0,8982 | -0,20653 | -0,84117 | -2,05413 |
| AF022072 | -1,2526 | -0,28443 | 0,5527 | -1,91293 | -3,6802 |
| BC025837 | 0,469433 | 0,380233 | -0,2976 | 0,9087 | 1,652233 |
| BG076151 | 0,602567 | 0,727967 | -0,12193 | 1,946333 | 2,237733 |
| BF719766 | 0,389167 | 0,9747 | -0,0442 | 1,823667 | 2,045133 |
| AV377066 | 1,0883 | 0,757233 | -0,65863 | 0,960533 | 2,462133 |
| BG065754 | 0,838267 | 0,8607 | 0,0569 | 0,958667 | 1,179133 |
| BC025501 | 1,140267 | 1,5389 | -0,1475 | 1,6386 | 2,022933 |
| NM_134188 | 0,012067 | 0,5856 | 0,596967 | -0,50007 | -0,06593 |
| NM_021451 | 1,100433 | 0,6364 | -0,7054 | 3,698367 | 2,681033 |
| BC026422 | 0,8843 | 0,6019 | -0,16643 | 2,220467 | 2,651367 |
| BC015270 | 1,092667 | 0,790767 | -0,13273 | 0,981567 | 0,633867 |
| NM_053168 | 1,268333 | 0,823033 | -0,26757 | 1,0583 | 1,4821 |
| BB027848 | -0,016 | 0,0761 | -0,252 | 1,126333 | 2,179833 |
| AV327248 | 0,805533 | 1,102433 | 0,0668 | 4,114233 | 4,258133 |
| AV219418 | 0,281667 | 0,278133 | -0,36947 | 2,743333 | 2,872767 |
| BG069873 | 0,727567 | 1,299367 | -0,27473 | 2,2987 | 2,174733 |
| AF204959 | 1,403933 | 0,3057 | 0,2152 | -0,29927 | 0,8169 |
| NM_030697 | 1,8143 | 1,4578 | -0,25197 | 3,210733 | 3,874367 |
| BM198879 | 1,610067 | 1,054033 | -0,2382 | 1,2634 | 2,011933 |
| AW543723 | 1,426867 | 1,993167 | 0,106467 | 1,950433 | 2,3145 |
| AK014608 | 1,764733 | 1,664767 | -0,09823 | 1,756333 | 2,3711 |
| AV298304 | 1,030433 | 0,6204 | -0,18307 | 1,8715 | 1,9706 |
| BC012260 | 1,2375 | 0,312733 | -0,64603 | 1,0548 | 2,236967 |
| NM_013866 | 0,951267 | 0,776033 | -0,13603 | 2,361133 | 2,300333 |
| AF065917 | 0,689633 | 0,922733 | -0,12417 | 1,803833 | 1,033367 |
| AF297615 | 1,1224 | 1,734833 | 0,031867 | 3,2188 | 2,358333 |
| BB770528 | 0,7477 | 0,697733 | -0,5052 | 2,799567 | 2,603367 |
| BC012247 | 0,367667 | 0,445067 | -0,08253 | 1,6567 | 2,093633 |
| NM_011316 | 0,2733 | 0,187133 | -0,3157 | 2,1585 | 2,611367 |
| NM_007987 | 0,627967 | 0,828667 | 0,069233 | 2,420733 | 2,6645 |
| BI660702 | 0,4068 | 1,3867 | 0,1219 | 2,513867 | 3,741633 |
| BM230508 | 0,591867 | 1,297 | 0,282033 | 2,303567 | 2,556067 |
| AI594683 | 1,047633 | 0,731 | -0,24517 | 3,163767 | 3,670633 |
| NM_011176 | 1,288967 | 1,507833 | -0,03387 | 1,574367 | 2,077667 |
| BB463610 | 1,7658 | 2,143967 | -0,37777 | 1,9965 | 2,535 |
| BC019882 | 0,4654 | 2,0133 | 1,3218 | 2,057333 | 3,446367 |
| AK007854 | 1,391233 | 0,3514 | -0,90083 | 2,172867 | 2,898333 |
| BC010462 | 0,998467 | 0,666267 | -0,14753 | 0,751867 | 1,162867 |
| BB043558 | 1,671433 | 1,246667 | -0,12047 | 2,406733 | 2,307633 |
| NM_008522 | 1,054933 | 1,113967 | 0,127667 | 3,616333 | 3,9908 |
| NM_012006 | 1,250567 | 3,254667 | 1,9403 | 2,032633 | 3,958567 |
| BB275142 | 1,009433 | 1,003367 | -0,02303 | 1,902233 | 1,944367 |
| BC008626 | 1,4158 | 0,823433 | -0,63527 | 2,035033 | 2,3005 |
| BI651416 | 1,8011 | 1,678867 | 0,155167 | 1,7218 | 2,482133 |
| AK005731 | 2,2462 | 2,015867 | 0,082767 | 5,486167 | 5,7645 |

**Table 6 Gene expression data at 24 hr with false GTX compounds**

| | | | | | |
|---|---|---|---|---|---|
| AK010447 | 0,060966667 | 0,496666667 | -0,121066667 | 0,0701 | 0,076966667 |
| BB318221 | -0,2464 | 0,0942 | 0,000233333 | -0,147933333 | 0,113766667 |
| BG261907 | -0,117733333 | 0,3643 | 0,092933333 | 0,3964 | -0,1486 |
| Y15910 | 0,066933333 | -0,539833333 | 0,065333333 | -0,310033333 | -0,2692 |
| AV095209 | 0,5754 | 0,907233333 | 0,316166667 | 0,8331 | 0,156433333 |
| AK019979 | 0,3731 | 0,938366667 | 0,113866667 | 0,250166667 | 0,084466667 |
| BC016073 | 0,030166667 | 0,362733333 | 0,0051 | 0,199266667 | -0,051833333 |
| BB821363 | -0,188333333 | -0,218966667 | -0,084 | -0,233766667 | 0,1765 |
| AI596632 | 0,225066667 | -0,496166667 | 0,1214 | -0,456033333 | -0,226966667 |
| AW986246 | 1,210366667 | 1,7055 | 0,016833333 | 1,388133333 | 0,319833333 |
| NM_028803 | 0,524233333 | 0,363333333 | -0,0826 | -0,250933333 | -0,045933333 |
| AV141095 | 0,181666667 | 0,178933333 | -0,129333333 | 0,285466667 | -0,070466667 |
| AF000969 | -0,100266667 | -0,683866667 | -0,12 | -0,575266667 | -0,371733333 |
| BB526605 | -0,213366667 | 1,641133333 | 0,340733333 | 0,096166667 | -0,014533333 |
| NM_008576 | 1,199266667 | 1,696333333 | 0,358566667 | 1,2925 | 0,106 |
| BG070887 | -0,067733333 | 0,343166667 | -0,051633333 | -0,2209 | -0,042733333 |
| AW543460 | 0,226333333 | 0,912666667 | 0,060366667 | 0,243666667 | -0,0366 |
| BC016265 | 0,0177 | 0,034333333 | 0,015066667 | 0,162166667 | -0,254666667 |
| AV223474 | -0,409433333 | -0,084366667 | -0,073466667 | -0,2496 | -0,069566667 |
| AI987929 | -0,100433333 | 2,650333333 | 0,6977 | 1,662633333 | 0,441433333 |
| AK009736 | 0,9644 | 1,582066667 | -0,0957 | 1,170733333 | 0,533733333 |
| AK007766 | -0,123533333 | -0,169666667 | -0,051666667 | -0,044633333 | -0,0226 |
| AK004419 | -0,120266667 | -0,0775 | -0,017933333 | -0,015366667 | 0,011133333 |
| AV173571 | -0,013666667 | -0,003133333 | -0,157433333 | -0,248433333 | 0,041966667 |
| BB308836 | -0,369233333 | 0,037633333 | -0,0317 | 0,1666 | 0,2558 |
| BC004827 | 0,365366667 | 0,539466667 | 0,393733333 | 0,9258 | -0,101133333 |
| AW240761 | 0,028033333 | 0,026966667 | 0,001666667 | -0,157033333 | -0,200633333 |
| BG066903 | 0,336833333 | 1,004933333 | 0,0241 | 0,250566667 | -0,1277 |
| NM_025770 | 0,344466667 | 1,050666667 | -0,012633333 | 0,266333333 | -0,1372 |
| BC025915 | 0,058266667 | 0,3343 | -0,053833333 | 0,317566667 | -0,026433333 |
| NM_018770 | 0,139966667 | 0,386766667 | -0,088533333 | 0,468866667 | 0,1391 |
| AF022072 | 0,621 | 0,848566667 | 0,4544 | 0,3385 | -0,4275 |
| BC025837 | -0,504233333 | -0,818133333 | -0,3033 | -0,3383 | -0,042266667 |
| BG076151 | 0,121266667 | -0,2933 | -0,086366667 | -0,136733333 | 0,072433333 |
| BF719766 | 0,0395 | -0,2859 | -0,115266667 | 0,159566667 | -0,193466667 |
| AV377066 | -0,6354 | -1,694833333 | -0,2482 | -0,4922 | -0,321166667 |
| BG065754 | 0,3287 | -0,250333333 | -0,089166667 | 0,482866667 | -0,2279 |
| BC025501 | 0,0663 | -0,2167 | -0,226066667 | 0,4589 | 0,197866667 |
| NM_134188 | 0,501033333 | -0,763733333 | 0,869566667 | 0,127533333 | -0,2881 |
| NM_021451 | 0,023566667 | -1,505 | 0,013033333 | -0,611033333 | -0,529466667 |
| BC026422 | -0,3825 | -0,8176 | 0,0232 | 0,280033333 | 0,287266667 |
| BC015270 | -0,230966667 | -0,7846 | 0,118566667 | -0,392366667 | -0,033533333 |
| NM_053168 | -0,067833333 | -0,1598 | -0,201 | -0,022566667 | -0,0493 |
| BB027848 | -0,571633333 | -1,7858 | -0,036966667 | -1,303666667 | -0,280733333 |
| AV327248 | 0,147666667 | -0,089166667 | 0,0212 | 0,311566667 | -0,082 |
| AV219418 | -0,796766667 | -1,032 | -0,2907 | -0,180833333 | 0,0363 |
| BG069873 | -0,1027 | -0,132566667 | -0,210733333 | 0,007866667 | -0,047633333 |
| AF204959 | -0,5019 | -2,025133333 | -0,280033333 | -1,232033333 | -0,417833333 |
| NM_030697 | -0,578533333 | 0,181633333 | -0,162333333 | 1,3142 | 0,220433333 |
| BM198879 | 0,293533333 | -0,328833333 | -0,2194 | 0,1402 | -0,006766667 |
| AW543723 | 0,148666667 | 0,670633333 | 0,294266667 | 0,419266667 | -0,0994 |
| AK014608 | 0,5726 | 0,295766667 | -0,176833333 | 0,032733333 | -0,122533333 |
| AV298304 | 0,1881 | -0,583933333 | -0,0861 | 0,2496 | -0,036066667 |
| BC012260 | -0,824033333 | 0,118933333 | -0,1838 | -0,168866667 | 0,254466667 |
| NM_013866 | -0,1346 | -0,280766667 | -0,195333333 | 0,065433333 | 0,058933333 |
| AF065917 | 0,012133333 | -0,457266667 | -0,202333333 | -0,102433333 | -0,210133333 |
| AF297615 | 0,069633333 | -0,279733333 | 0,031033333 | 0,804666667 | -0,0684 |
| BB770528 | -0,327066667 | -1,122266667 | -0,083266667 | -0,310933333 | 0,138566667 |
| BC012247 | -0,326066667 | -1,499833333 | -0,0311 | -0,2845 | -0,0122 |
| NM_011316 | -0,044633333 | -0,590933333 | -0,1988 | -0,131366667 | 0,0831 |
| NM_007987 | 0,231266667 | -1,231166667 | -0,133733333 | 0,174366667 | -0,055233333 |
| BI660702 | 0,081666667 | -0,608966667 | -0,153866667 | -0,058966667 | 0,051133333 |
| BM230508 | 0,3615 | -0,6065 | -0,1178 | 0,3449 | 0,034966667 |
| AI594683 | -0,315666667 | -0,351733333 | -0,216266667 | -0,2712 | -0,097833333 |
| NM_011176 | -0,053933333 | -0,571666667 | -0,144533333 | 0,591366667 | 0,057833333 |
| BB463610 | 0,384833333 | 0,210633333 | -0,3672 | -0,1026 | -0,2188 |
| BC019882 | -0,0399 | -1,259166667 | 1,0984 | 0,712366667 | 0,0605 |
| AK007854 | -0,4492 | -1,735733333 | -0,428266667 | -0,9336 | -0,076 |
| BC010462 | -0,589533333 | -0,865433333 | -0,408866667 | -0,5852 | 0,056633333 |
| BB043558 | 0,494633333 | -0,415933333 | -0,129166667 | 0,343133333 | -0,373433333 |
| NM_008522 | -0,358133333 | -0,301766667 | 0,0771 | -0,095933333 | -0,059633333 |
| NM_012006 | 0,6012 | -1,4501 | 1,861966667 | 1,0762 | -0,253166667 |
| BB275142 | -0,195366667 | -0,418433333 | 0,028233333 | -0,500433333 | -0,025066667 |
| BC008626 | -0,242 | -2,247633333 | -0,270533333 | -0,372466667 | -0,4422 |
| BI651416 | 0,052366667 | -0,0339 | 0,013866667 | 0,265266667 | 0,136366667 |
| AK005731 | 0,196466667 | -0,511133333 | -0,173333333 | 0,6919 | 0,114266667 |

**Table 7 Gene expression data at 48 hr with true GTX compounds**

| **GENEBANK ACCESS CODE** | **AFB1** | **BaP** | **2-AAF** | **DMN** | **MitC** |
|---|---|---|---|---|---|
| | **48 h** | **48 h** | **48 h** | **48 h** | **48 h** |
| | **average** | **average** | **average** | **average** | **average** |
| AK005731 | 2,2191 | 3,0498 | 0,011133333 | 4,616833333 | 6,088666667 |
| BI651416 | 1,978966667 | 1,9573 | 0,350166667 | 1,8588 | 2,2069 |
| NM_008522 | 2,271133333 | 2,9351 | 0,1967 | 4,993466667 | 5,9249 |
| BB043558 | 1,629366667 | 1,692466667 | 0,195966667 | 2,186833333 | 2,331833333 |
| NM_007987 | 0,877166667 | 1,352666667 | 0,515966667 | 1,665166667 | 2,2406 |
| BC022148 | 1,0426 | 1,323566667 | 0,456033333 | 1,8449 | 2,668233333 |
| BC019882 | 1,2329 | 2,160033333 | 1,576933333 | 1,494 | 2,441633333 |
| BB463610 | 1,2472 | 1,672366667 | 0,0872 | 1,546 | 2,727766667 |
| BM230508 | 0,637 | 1,158366667 | 0,0745 | 1,539833333 | 2,570533333 |
| AI594683 | 1,508466667 | 1,465666667 | -0,052133333 | 3,7761 | 4,611033333 |
| AV327248 | 1,4639 | 2,067166667 | -0,091766667 | 4,022733333 | 4,918266667 |
| BE956581 | 1,7703 | 1,451433333 | 0,1808 | 3,032866667 | 3,679666667 |
| NM_011176 | 1,3047 | 1,4287 | -0,263233333 | 1,597133333 | 1,856466667 |
| BM200015 | 0,934166667 | 1,0174 | 0,512666667 | 1,160333333 | 1,702233333 |
| BB223872 | 0,526333333 | 1,1634 | 0,321566667 | 1,694233333 | 2,200766667 |
| AF297615 | 2,175266667 | 1,571833333 | -0,679133333 | 3,153166667 | 1,840933333 |
| BC027026 | 0,6995 | 1,378666667 | 0,806333333 | 2,570766667 | 2,873 |
| NM_012006 | 1,7541 | 2,867133333 | 2,770866667 | 1,6671 | 3,166133333 |
| AK014608 | 1,185766667 | 1,245366667 | -0,019866667 | 1,390533333 | 2,8746 |
| BC012247 | 0,7954 | 1,476733333 | 0,6286 | 1,669566667 | 2,2674 |
| BC027121 | 0,979866667 | 1,345266667 | -0,134133333 | 2,074533333 | 2,741666667 |
| BG797099 | 1,027266667 | 1,8809 | -0,061733333 | 1,703866667 | 2,6244 |
| BB743970 | 0,381333333 | 1,510133333 | 0,766433333 | 3,154366667 | 3,5588 |
| BF719766 | 1,1094 | 0,950666667 | 0,265166667 | 1,2016 | 2,112066667 |
| BC027185 | 0,0776 | 0,708133333 | 0,4588 | 1,461733333 | 2,115066667 |
| AF033112 | 1,032833333 | 1,4254 | -0,039466667 | 1,816933333 | 2,218866667 |
| BG065754 | 0,688433333 | 0,974633333 | 0,373833333 | 0,995333333 | 1,3079 |
| BB781615 | 0,972233333 | 0,927133333 | -0,116433333 | 1,486966667 | 0,895166667 |
| BC013893 | 0,3479 | 1,040066667 | 0,493633333 | 0,548733333 | 1,863933333 |
| BC003284 | 0,703633333 | 0,731333333 | 0,025633333 | 1,379133333 | 2,092633333 |
| BC006713 | 0,941966667 | 0,4721 | 0,4411 | 1,668066667 | 1,2958 |
| NM_011075 | 1,1557 | 1,197733333 | -0,5522 | 2,401 | 3,096466667 |
| BB009155 | 1,3686 | 0,784 | -0,5851 | 2,342966667 | 2,9201 |
| BG967046 | 0,624866667 | 0,593566667 | 0,0391 | 1,1973 | 1,305133333 |
| NM_030697 | 1,270633333 | 1,3687 | -0,478333333 | 2,5583 | 3,958066667 |
| BB275142 | 0,5302 | 1,0559 | 0,110166667 | 1,161633333 | 2,281633333 |
| AV246296 | 1,097933333 | 1,1524 | -0,54 | 0,957233333 | 1,4788 |
| NM_013738 | 0,4146 | 0,9012 | -0,239566667 | 1,562466667 | 2,749033333 |
| NM_018881 | 0,200133333 | 3,369333333 | 0,906066667 | 0,948066667 | 2,279366667 |
| BM936480 | 0,1463 | 3,0213 | 0,916866667 | 0,778066667 | 2,006033333 |
| BM198879 | 1,579166667 | 0,808866667 | -0,288733333 | 1,096266667 | 1,927533333 |
| AK018383 | 0,406933333 | 1,273266667 | 0,230266667 | 0,824633333 | 0,994 |
| AV254764 | 1,542533333 | 1,480466667 | -0,006466667 | 1,133466667 | 1,411166667 |
| BC021352 | 1,590266667 | 0,705233333 | -1,126533333 | 3,512033333 | 3,121333333 |
| BB027848 | -0,004366667 | 0,473766667 | 0,5378 | 1,190666667 | 1,879 |
| AK017734 | 0,4796 | 0,7529 | 0,0451 | 1,0611 | 1,834766667 |
| AF069954 | 0,2361 | 0,8863 | 0,351766667 | 1,631166667 | 2,259766667 |
| BB770528 | 0,867766667 | 0,7877 | -0,5594 | 1,709333333 | 2,0332 |
| NM_009897 | 1,053566667 | 1,182233333 | -0,003733333 | 3,618966667 | 4,658266667 |
| AK007854 | 1,5416 | 1,6125 | 1,4303 | 0,839633333 | 1,500233333 |
| BI966443 | 0,698966667 | 1,3186 | 0,1201 | 3,294933333 | 4,094033333 |
| NM_013929 | 0,744866667 | 1,061633333 | -0,041633333 | 1,890866667 | 2,481333333 |
| BG076151 | 0,477866667 | 0,759933333 | -0,3358 | 1,205166667 | 2,5119 |
| AV251625 | 0,547133333 | 1,730566667 | 0,071033333 | 1,246066667 | 2,476566667 |
| AV219418 | 0,8603 | 1,558433333 | 0,783933333 | 4,5473 | 4,167766667 |
| NM_011316 | 0,6494 | 0,873466667 | 0,4369 | 2,0898 | 2,255733333 |
| NM_007980 | 0,411966667 | 1,752833333 | 1,503166667 | 0,325166667 | 2,9391 |
| BB046347 | 0,5079 | 0,7766 | 0,217766667 | 0,774333333 | 1,349533333 |
| AF335325 | 1,3645 | 1,4875 | -0,308833333 | 1,6857 | 1,754566667 |
| AK010738 | 0,6579 | 0,911166667 | 0,1851 | 1,342566667 | 1,979733333 |
| NM_134188 | -0,158966667 | 0,288366667 | 0,908766667 | -0,637766667 | 0,4786 |
| NM_008935 | -1,772 | -1,4799 | 0,027833333 | -1,382066667 | -3,600866667 |
| BB140436 | -0,4938 | -0,457233333 | 0,469333333 | -1,281166667 | -1,601366667 |
| NM_019738 | -2,369733333 | -2,585566667 | -0,193033333 | -2,9169 | -2,771766667 |
| X62701 | -0,661133333 | -1,2338 | -0,356033333 | 0,071566667 | -1,3159 |
| AV141095 | -1,192433333 | -1,4056 | -0,582933333 | -0,866333333 | -2,411233333 |
| AI747296 | -1,533166667 | -2,124066667 | -0,500266667 | -0,433266667 | -2,056266667 |
| BC005552 | -0,7309 | -0,212133333 | -0,125966667 | -0,523533333 | -1,341933333 |
| BB458460 | -1,131533333 | -1,384133333 | -0,336766667 | -0,693433333 | -2,7207 |
| BG076333 | -0,3573 | -0,409133333 | -0,130566667 | -0,799466667 | -1,870033333 |
| AK019979 | -2,270633333 | -1,0966 | -0,509866667 | -1,110233333 | -3,757366667 |
| AV095209 | -0,6169 | -0,765433333 | -0,371833333 | -0,453966667 | -2,780333333 |
| AV216768 | -1,281733333 | -1,446966667 | -0,6814 | -0,2641 | -4,148766667 |
| AV221299 | -0,787333333 | -1,168933333 | 0,6759 | -1,5693 | -3,709666667 |
| BQ174991 | -0,6262 | -1,407933333 | -0,342633333 | -0,28 | -2,777066667 |
| NM_013642 | -0,252766667 | -1,442166667 | -0,653466667 | 0,3461 | -0,784533333 |
| L21027 | -1,867666667 | -1,779266667 | -0,728966667 | -0,4472 | -4,611766667 |
| BB204486 | -1,371333333 | -1,5693 | -0,6445 | -0,3108 | -4,143533333 |
| BC025169 | -1,029533333 | -0,511366667 | -0,222233333 | -1,295533333 | -3,296166667 |
| BC026131 | -0,447566667 | -1,045333333 | -0,3489 | -0,7571 | -0,998633333 |
| BC010318 | -0,956533333 | -0,552866667 | -0,022133333 | -1,166866667 | -1,728666667 |
| BB730977 | -0,256 | 0,421766667 | -0,993666667 | -0,656 | -2,360033333 |
| AA561726 | -1,480766667 | -1,7432 | -0,7419 | -0,353433333 | -4,371633333 |
| BC012955 | -1,402833333 | -0,944466667 | 0,050733333 | -1,970133333 | -2,240533333 |
| BC004827 | -1,254533333 | -1,186033333 | -0,2015 | -1,0689 | -3,638633333 |
| NM_007556 | -0,9667 | -1,089 | 0,027033333 | -1,289533333 | -4,012333333 |
| NM_134147 | -1,510133333 | -1,2396 | 0,042133333 | -1,579366667 | -2,8471 |
| AV173869 | -0,881433333 | -1,923533333 | -0,3782 | -1,271666667 | -2,2889 |
| AF022072 | -0,991333333 | -1,017566667 | 0,0482 | -1,2797 | -3,369966667 |
| BC019379 | -1,263466667 | -1,9807 | -1,214833333 | -0,360166667 | -2,7493 |
| AK010447 | -1,2768 | -1,207066667 | -0,3256 | -0,863433333 | -1,6388 |
| BC017615 | -2,585433333 | -2,233833333 | -0,522933333 | -2,7334 | -3,946966667 |
| BB246912 | -1,2439 | -1,539866667 | -0,226366667 | -0,980566667 | -1,866433333 |
| AF000969 | -2,345533333 | -2,251433333 | -0,831866667 | -1,113833333 | -3,3606 |
| BG066491 | -1,6995 | -2,097833333 | -1,541033333 | -0,975666667 | -1,928533333 |
| AF055573 | -2,3744 | -1,5573 | -0,289466667 | -1,912533333 | -2,915766667 |
| NM_053122 | -2,304433333 | -1,795033333 | -0,105966667 | -2,3248 | -4,049166667 |

**Table 8 Gene expression data at 48 hr with false GTX compounds**

| **GENEBANK ACCESS CODE** | **2-CP** | **4-NP** | **ANAC** | **Q** | **8Q** |
|---|---|---|---|---|---|
| | **48 h** | **48 h** | **48 h** | **48 h** | **48 h** |
| | **average** | **average** | **average** | **average** | **average** |
| AK005731 | -0,6019 | -0,9581 | -0,649766667 | -0,8953 | -0,039966667 |
| BI651416 | -0,087533333 | -0,028733333 | -0,105066667 | -0,498166667 | 0,067766667 |
| NM_008522 | 0,0213 | -0,239266667 | -0,069566667 | -0,039166667 | 0,0486 |
| BB043558 | -0,229266667 | -0,273633333 | -0,188 | 0,038166667 | -0,234666667 |
| NM_007987 | -0,3447 | -0,8469 | -0,005833333 | -0,073433333 | 0,034866667 |
| BC022148 | 0,191933333 | -1,1322 | -0,1976 | 0,2323 | 0,044966667 |
| BC019882 | 0,814833333 | -2,4942 | 0,194833333 | -0,021766667 | -0,171866667 |
| BB463610 | 0,0113 | -0,037266667 | -0,218566667 | -0,254 | -0,236866667 |
| BM230508 | -0,2554 | -0,973133333 | -0,470966667 | -0,670733333 | 0,076033333 |
| AI594683 | -0,2197 | -0,4763 | -0,522366667 | -0,1952 | 0,123533333 |
| AV327248 | -0,221533333 | -0,014933333 | 0,0856 | -0,184166667 | 0,117633333 |
| BE956581 | 0,0444 | 0,0346 | 0,042666667 | 0,106333333 | 0,403033333 |
| NM_011176 | -0,0046 | -1,635266667 | -0,460666667 | -0,357866667 | 0,1358 |
| BM200015 | -0,3607 | -0,4429 | 0,009533333 | -0,2469 | 0,080866667 |
| BB223872 | 0,174333333 | -0,653566667 | -0,428733333 | -0,240866667 | 0,057533333 |
| AF297615 | -0,5906 | -1,352433333 | 0,111 | -0,029433333 | -0,009333333 |
| BC027026 | -0,217666667 | 0,052733333 | -0,007933333 | 0,363633333 | 0,2479 |
| NM_012006 | -0,1277 | -0,006733333 | 1,5971 | 0,737333333 | 0,580566667 |
| AK014608 | -0,012633333 | -0,307233333 | -0,455 | -0,318133333 | -0,2447 |
| BC012247 | 0,522066667 | -0,635466667 | -0,5319 | 0,185266667 | -0,006933333 |
| BC027121 | -0,6553 | -0,2406 | -0,1424 | 0,003766667 | -0,1207 |
| BG797099 | 0,004233333 | -0,066966667 | -0,020966667 | -0,318533333 | 0,0658 |
| BB743970 | -0,168566667 | -0,1636 | 0,155133333 | -0,154266667 | 0,1936 |
| BF719766 | -0,125866667 | -0,5024 | 0,076366667 | 0,168333333 | -0,271666667 |
| BC027185 | 0,062733333 | -1,381733333 | -0,238133333 | -0,431666667 | -0,120933333 |
| AF033112 | -0,665066667 | 0,151966667 | 0,142233333 | 0,025933333 | -0,017033333 |
| BG065754 | -0,2817 | -0,338066667 | 0,0641 | -0,269166667 | -0,0909 |
| BB781615 | 0,007033333 | -0,750133333 | -0,293733333 | -0,302466667 | -0,006366667 |
| BC013893 | 0,487133333 | -1,496 | -0,5709 | -0,5246 | -0,165433333 |
| BC003284 | -0,239466667 | -0,587633333 | -0,210833333 | -0,3991 | 0,09 |
| BC006713 | 0,2405 | -0,7469 | -0,130466667 | 0,141933333 | -0,103533333 |
| NM_011075 | -0,578166667 | -1,007866667 | 0,389133333 | -0,8714 | 0,161766667 |
| BB009155 | -0,348033333 | -0,6079 | -0,4697 | -0,644766667 | 0,006466667 |
| BG967046 | -0,155666667 | -0,632366667 | -0,256166667 | -0,2263 | -0,092566667 |
| NM_030697 | -0,0882 | -0,3339 | -0,6238 | 0,085966667 | -0,0261 |
| BB275142 | 0,091 | -0,196266667 | -0,287366667 | -0,530666667 | 0,0112 |
| AV246296 | -0,355833333 | -1,045766667 | -0,1054 | -0,449 | -0,113566667 |
| NM_013738 | -0,373033333 | -1,171233333 | -0,3378 | -0,4434 | -0,304966667 |
| NM_018881 | -0,0599 | -0,2965 | 0,083833333 | 0,266733333 | -0,167866667 |
| BM936480 | -0,074166667 | -0,411966667 | -0,043233333 | 0,1682 | 0,0483 |
| BM198879 | -0,102566667 | -0,421266667 | -0,160833333 | -0,138233333 | -0,056166667 |
| AK018383 | -0,215766667 | -0,500633333 | -0,0203 | -0,063433333 | 0,0679 |
| AV254764 | 0,396 | -0,449933333 | -0,264966667 | -0,091133333 | 0,024033333 |
| BC021352 | -0,719166667 | -1,279566667 | 0,065633333 | -1,142833333 | 0,472066667 |
| BB027848 | 0,205366667 | -1,954166667 | -0,2609 | -0,5634 | -0,066066667 |
| AK017734 | 0,148866667 | -1,2326 | -0,527466667 | -0,223133333 | 0,0878 |
| AF069954 | 0,199 | -0,442866667 | -0,384666667 | -0,168 | 0,184133333 |
| BB770528 | -0,4836 | -0,9985 | -0,001733333 | -0,5238 | -0,018233333 |
| NM_009897 | -0,061033333 | -0,019033333 | 0,099633333 | 0,246833333 | 0,111033333 |
| AK007854 | 0,551333333 | -0,141066667 | -0,224233333 | 1,0645 | 0,059733333 |
| BI966443 | -0,017133333 | 0,2259 | 0,077233333 | 0,1621 | 0,133533333 |
| NM_013929 | -0,529133333 | 0,175266667 | -0,006633333 | 0,092566667 | 0,033 |
| BG076151 | -0,0769 | -0,657266667 | -0,240966667 | -0,7006 | -0,188666667 |
| AV251625 | 0,067233333 | -0,1615 | 0,0423 | -0,2405 | 0,038133333 |
| AV219418 | 0,828066667 | 0,6295 | 0,153866667 | 0,7223 | 0,4492 |
| NM_011316 | 0,582866667 | -0,9295 | -0,471733333 | -0,248533333 | 0,063766667 |
| NM_007980 | -0,284766667 | -1,581066667 | 0,177566667 | 1,050633333 | -0,506433333 |
| BB046347 | 0,187 | -0,804466667 | -0,107166667 | 0,374733333 | 0,1608 |
| AF335325 | -0,056666667 | 0,507133333 | -0,0865 | -0,3547 | 0,141766667 |
| AK010738 | 0,0059 | -0,156866667 | -0,066866667 | 0,034566667 | 0,3608 |
| NM_134188 | -0,137266667 | -0,440166667 | 0,238166667 | 0,577266667 | -0,070666667 |
| NM_008935 | -0,212433333 | -1,001666667 | 0,377866667 | 0,588066667 | 0,408166667 |
| BB140436 | 0,2823 | 2,071 | -0,072366667 | 0,455033333 | 0,286066667 |
| NM_019738 | -0,514266667 | -1,132266667 | -0,632566667 | -0,7691 | -0,9543 |
| X62701 | -0,146033333 | 0,7376 | 0,7542 | 0,842633333 | 0,154 |
| AV141095 | -0,4102 | -0,105133333 | -0,0311 | -0,035733333 | -0,172533333 |
| AI747296 | -0,263733333 | -0,3288 | -0,0394 | -0,157233333 | -0,000966667 |
| BC005552 | 0,125466667 | 0,4521 | 0,305566667 | 0,365 | 0,134333333 |
| BB458460 | -0,179166667 | 0,164466667 | 0,007966667 | -0,250466667 | 0,131733333 |
| BG076333 | 0,4759 | 0,873133333 | 0,238266667 | 0,256933333 | -0,053333333 |
| AK019979 | -0,424933333 | 0,040866667 | 0,045466667 | -0,645633333 | -0,215433333 |
| AV095209 | -0,034666667 | 0,6413 | 0,2956 | 0,0573 | 0,260166667 |
| AV216768 | -0,417933333 | -0,004066667 | 0,415333333 | 0,4269 | 0,167866667 |
| AV221299 | 0,454466667 | 0,7477 | 0,164933333 | 0,3941 | 0,1454 |
| BQ 174991 | 0,1864 | 0,401733333 | 0,4039 | 0,543166667 | -0,1546 |
| NM_013642 | 0,3967 | 1,007033333 | 0,328566667 | 0,7153 | 0,265866667 |
| L21027 | -0,5404 | -0,030733333 | 0,3857 | 0,4391 | 0,2905 |
| BB204486 | -0,3875 | -0,0837 | 0,3188 | 0,321133333 | 0,130466667 |
| BC025169 | 0,928266667 | 0,371166667 | -0,0788 | 0,176 | 0,022133333 |
| BC026131 | 0,4079 | 0,212366667 | 0,0392 | 0,3886 | -0,0852 |
| BC010318 | 0,1986 | 0,1861 | 0,135333333 | 0,227366667 | 0,0565 |
| BB730977 | -0,7313 | 0,991966667 | 0,753233333 | 0,126966667 | 0,329766667 |
| AA561726 | -0,429733333 | -0,08 | 0,319366667 | 0,347333333 | 0,173933333 |
| BC012955 | 0,2863 | 0,105133333 | -0,255433333 | 0,249966667 | -0,304933333 |
| BC004827 | -0,247666667 | -0,2247 | 0,375566667 | 0,383233333 | -0,070433333 |
| NM_007556 | 0,168533333 | 0,427366667 | 0,524666667 | 0,467266667 | 0,0638 |
| NM_134147 | 0,6449 | 0,014333333 | -0,220733333 | -0,2048 | -0,1757 |
| AV173869 | 0,3109 | 0,1411 | -0,005066667 | -0,3593 | -0,025566667 |
| AF022072 | 0,040666667 | 0,606166667 | 0,5563 | 0,7799 | 0,0814 |
| BC019379 | -0,192566667 | -0,5665 | 0,0072 | -0,033133333 | -0,155466667 |
| AK010447 | -0,050233333 | 0,595633333 | -0,108333333 | -0,127666667 | 0,062233333 |
| BC017615 | -0,3193 | -1,535366667 | 0,068266667 | -0,278633333 | -0,4904 |
| BB246912 | 0,5739 | 0,302566667 | -0,1992 | 0,039433333 | 0,3325 |
| AF000969 | 0,294433333 | -0,820166667 | -0,559933333 | 0,6762 | -0,180933333 |
| BG066491 | -0,348866667 | -0,8219 | -0,061 | -0,627766667 | -0,177133333 |
| AF055573 | 0,368566667 | -0,197333333 | -0,168666667 | 0,0039 | -0,094566667 |
| NM_053122 | 0,1396 | -0,096466667 | -0,045133333 | -0,1158 | -0,142033333 |

### Examples

### Example 1: materials used

Dulbecco's modified Eagle's medium (DMEM), fetal calf serum (FCS), Hanks' calcium- and magnesium-free buffer, insulin and Trizol were obtained from Invitrogen (Breda, The Netherlands). Glucagon, hydrocortisone, collagenase type IV, Benzo(a)pyrene (BaP), Aflatoxin B1 (AFB1), 2-Acetylaminofluorene (2-AAF), Dimethylnitrosamine (DMN), Mitomycin C (MitC), o-Anthranilic acid (ANAC), 2-(Chloromethyl)pyridine.HCl (2-CP), 4-Nitro-o-phenylenediamine (4-NP), Quercetin (Q), 8-Hydroxyquinoline (8-HQ), Trypan blue, dimethylsulphoxide (DMSO), bovine serum albumin (BSA), 4',6-diamidino-2-phenylindole (DAPI) and Tween-20 were purchased from Sigma-Aldrich (Zwijndrecht, The Netherlands). Triton X-100, NaCl, Na₂HPO₄.2H₂O and NaH₂PO₄ were obtained from Merck (Darmstadt, Germany) and paraformaldehyde from ICN biomedicals (Auroro, Ohio). Collagen Type I Rat Tail was obtained from BD BioSciences (Bedford, MA). The RNeasy minikit was obtained from Qiagen, Westburg B.V. (Leusden, The Netherlands). The 5x MegaScript T7 Kit was obtained from Ambion (Austin, TX). The GeneChip^{®} Expression 3'-Amplification Two-Cycle cDNA Synthesis Kit and Reagents, the Hybridization, Wash and Stain Kit and the Mouse Genome 430 2.0 Arrays were purchased from Affymetrix (Santa Clara, CA).

### Example 2: Animals

Permission for performing animal studies was obtained from the Animal Ethical Committee. Adult male C57/B6 mice (Charles River), weighing 20-25 g, were obtained from Charles River GmbH, Sulzfeld, Germany. This mouse strain was chosen because it is frequently used in toxicological and pharmacological investigations, and it is a common background for transgenic mouse strains. The animals were housed in macrolon cages with sawdust bedding at 22°C and 50-60% humidity. The light cycle was 12 h light/12 h dark. Feed and tap water were available *ad libitum.*

### Example 3: isolation of hepatocytes.

Hepatocytes were isolated from adult male C57/B6 mice by a two-step collagenase perfusion method according to Seglen and Casciano (16, 17), with modifications as described before (18). Cell viability and yield were determined by trypan blue exclusion.

### Example 4: Cell culturing and treatments.

Cells with viability >85%, were cultured in a collagen-collagen sandwich formation as described before (18, 19, 20). Prior to treatment, primary cultures of mouse hepatocytes were allowed to recover for 40-42 h at 37°C in a humidified chamber with 95%/5% air/CO₂ in serum-free culture medium supplemented with insulin 0.5U/mi), glucagon (7 nanog/ml), hydrocortisone (7.5 microg/ml) and 2% penicillin/streptomycin (5000 U/ml penicillin; 5000 microM/ml streptomycin). Culture medium was refreshed every 24 h. After the recovery period, the culture medium was replaced by culture medium containing one of the selected ten compounds, or with vehicle control. Only non-cytotoxic doses were used for each compound, which were determined by the MTT assay (ca 80% viability) and are presented in Table 9. Cells were incubated for 24 or 48 h before being harvested for RNA isolation by adding Trizol reagent. Three independent replicate biological experiments with hepatocytes from different mice were conducted for each compound.

**Table 9 Solvents and dose used for several true GTX and false GTX compounds.**

| **Chemical** | **Solvent and dose (v/v %)** | **Dose** | **GTX in vitro** | **GTX in vivo** |
|---|---|---|---|---|
| | | | | |

| *True GTX compounds* | | | | |
|---|---|---|---|---|
| Benzo(a)pyrene | DMSO, 0.5 % | 30 µM | + | + |
| Aflatoxin B1 | DMSO, 0.5 % | 15 µM | + | + |
| 2-Acetylaminofluorene | DMSO, 0.5 % | 125 µM | + | + |
| Dimethylnitrosamine | | 2 mM | + | + |
| Mitomycin C | Ethanol, 0.5 % | 5 µM | + | + |
| | | | | |

| *False GTX compounds* | | | | |
|---|---|---|---|---|
| o-Anthranilic acid | DMSO, 0.5 % | 2 mM | + | - |
| 2-(Chloromethyl)pyridine.HCl | DMSO, 0.5 % | 125 µM | + | - |
| 4-Nitro-o-phenylenediamine | DMSO, 0.5 % | 2 mM | + | - |
| Quercetin | DMSO, 0.5 % | 200 µM | + | - |
| 8-Hydroxyquinoline | Ethanol, 0.5 % | 150 µM | + | - |

### Example 5: RNA isolation.

Total RNA was isolated from cultured mouse hepatocytes using Trizol and by means of the RNeasy kit according to the manufacturer's protocol. RNA concentrations were measured by means of a spectrophotometer and the quality of each RNA preparation was determined by means of a bio-analyzer (Agilent Technologies, The Netherlands). Only samples with a good quality (clear 18S and 28S peaks and RIN>6) were used for hybridization. Extracted RNA was stored at -80°C until further analysis.

### Example 6: Gene expression analysis, target preparation and hybridization.

Targets were prepared according to the Affymetrix protocol. cRNA targets were hybridized according to the manufacturer's recommended procedures on high-density oligonucleotide gene chips (Affymetrix Mouse Genome 430 2.0 GeneChip arrays). The gene chips were washed and stained using an Affymetrix fluidics station and scanned by means of an Affymetrix GeneArray scanner.

A total of eighty-two GeneChips was run. Normalization quality controls, including scaling factors, average intensities, present calls, background intensities, noise, and raw Q values, were within acceptable limits for all chips. Hybridization controls BioB, BioC, BioD, and CreX, were identified on all chips and yielded the expected increases in intensities.

### Example 7: Selection of differentially expressed probe sets; true versus false GTX.

Eighty-two datasets were obtained from this experiment. Raw data were imported into ArrayTrack (22, 23) and normalized using Robust Multi-array Average (RMA, integrated into ArrayTrack) (24).

Present-Marginal-Absent calls were used to identify and omit probe sets of poor quality (25). Subsequently, the remaining probe sets were logarithmically (base 2) transformed, corrected for vehicle control, and subjected to statistical analysis (24 h: 26100; 48 h: 26690; total: 27363). For each time point, probe sets were then selected for which expression was up- or down-regulated by at least one compound at a minimum of 1.2-fold in at least two out of three experiments with expressions altered in the same direction in all replicate and with a mean fold up- or down-regulated of 1.5 (26). The generated list with differentially expressed probe sets (log 2 ratios) was used for hierarchical clustering (HCA) and prediction analysis of microarray (PAM) (10776 probe sets at 24 h and 12180 probe sets at 48 h).

### Example 8: Class prediction and functional analysis; true versus false GTX

The software tool "prediction analysis of microarray" (PAM) was used for discriminating true GTX compounds from false GTX compounds (27). PAM uses gene expression data to calculate the shrunken centroid for each class and identifies the specific genes that determine the centroid most. Based on the nearest shrunken centroid, PAM is also capable of predicting to which class an unknown sample belongs (27). Class prediction was performed after 24 h and 48 h of exposure.

For this analysis, the gene list with differentially expressed probe sets was used. For each exposure period, 3 sets of genes (classifiers) were generated by PAM, using all ten treatments, based on the smallest estimated misclassification error rate (generated by 10-fold cross-validation) and a > 80% predicted test probability. This was done by using 2 experiments as training set and the third experiment for validation. This was done for all 3 possible combinations, each time leaving out another experiment. For each time point, the classifiers that were in common between the three training sets, were set as the final classifier set for that time point

### Example 9: Selection of differentially expressed genes: GTX vs non-GTX

Ninety datasets were obtained from this experiment. Raw data were normalized using Robust Multi-array Average (RMA) (24), using the custom chip description files (CDFs) as described by de Leeuw et al (BMC.Res.Notes, 2008, 1: 66.). Of the hybrid probe-set definitions included in the custom annotation, only the 16331 probe sets selected according to Dai et al (Nucleic Acids Res., 2005, 33: e175.) and the 4648 Affymetrix probe sets corresponding to an Entrez Gene ID were used in further analysis, giving a total of 20979 probe sets.

Subsequently, the remaining probe sets were logarithmically (base 2) transformed, corrected for vehicle control, and subjected to statistical analysis. For each gene, a significant response was scored if both of the following criteria were met: (a) if the gene expression values for the replicate compound-exposed samples differed significantly from the vehicle-exposed samples with a t-test p-value < 0.01; (b) if the average gene expression value for the replicate compound-exposed samples was at least twice that of the average vehicle-exposed samples. If none or only one of these criteria were met, no point was scored. These calculations were performed in the statistical package R.
The four genes with the highest scores in the GTX group and no scores in the non-GTX group were set as the classifier set.

### Example 10: Class prediction and functional analysis: GTX vs non-GTX

For this analysis, the same gene scoring system as described above was used for the four genes with the highest scores (1700007K13RIK, GAS2L3, SPC25, DDIT4L). Compounds were scored using these four genes and it was found that a positive score in at least one gene resulted in identification of GTX compounds and not for non-GTX compounds.

The validity of this approach was verified using a leave-one compound-out strategy, each time leaving out another compound, giving 80% prediction or better.

### Example 11: Validation of classifiers

For the purpose of validating the class discrimination models with the final classifier sets, gene expression data were generated for two additional true GTX compounds, phenacetin and DMBA, and for three False GTX compounds, cur, ethylacrylate and resorcinol and the vehicle control for exposure periods of 24 and 48 h. All the independent triplicate treatments of all compounds were classified correctly with a predicted test probability of 100% at both time points, with the exception of phenacetin, which is misclassified as a False GTX compound, only at 48 h (Table III below). This resulted in a positive prediction value of 100% for both time points and a negative prediction value of 89 and 80% for 24 and 48 h, respectively.

**Table 10 Overview of the five extra compounds used in primary mouse hepatocyte exposure validation study for true and false GTX prediction**

| **Chemical** | **Abbreviation** | **CAS nr.** | **Concentration** | **Vehicle** |
|---|---|---|---|---|
| | | | | |

| *True GTX compounds* | | | | |
|---|---|---|---|---|
| Dimethylbenzanthracene | DMBA | 57-97-6 | 500 µM | DMSO |
| Phenacetin | Phén | 62-44-2 | 1.5 mM | Ethanol |
| | | | | |

| *False GTX compounds* | | | | |
|---|---|---|---|---|
| Curcumin | Cur | 458-37-7 | 80 µM | DMSO |
| Ethyl acrylate | Ethylacrylate | 140-88-5 | 500 µM | Ethanol |
| Resorcinol | Resorcinol | 108-46-3 | 2 mM | Ethanol |

| **Table III.** Validation of the class prediction model with five additional compounds | | | | | | | |
|---|---|---|---|---|---|---|---|
| Compound | Genotoxic class | Prediction | | | | | |
| | | 24 h | | | 48 h | | |
| | | Exp 1 | Exp 2 | Exp 3 | Exp 1 | Exp 2 | Exp 3 |
| DMBA | GTX | GTX | GTX | GTX | GTX | GTX | GTX |
| Phen | GTX | GTX | GTX | GTX | FP-GTX | FP-GTX | FP-GTX |
| Cur | FP-GTX | FP-GTX | FP-GTX | FP-GTX | FP-GTX | FT-GTX | FP-GTX |
| Ethylacrylate | FP-GTX | FP-GTX | FP-GTX | FP-GTX | FP-GTX | FP-GTX | FP-GTX |
| Resorcinol | FP-GTX | FP-GTX | FP-GTX | FT-GTX | FP-GTX | FP-GTX | FP-GTX |

| | | | | | | | |
|---|---|---|---|---|---|---|---|
| The intersection of the classifiers from Table II, for each time point separately, was used for generating the classification model in PAM. The five new compounds were used for validating that model. | | | | | | | |

### REFERENCES

1. Dambach, et al., Toxicol Pathol, 2005. 33(1): p. 17-26.
2. Tsujimura, K., et al., Cancer Sci, 2006. 97(10): p. 1002-10.
3. Kirkland, D., et al., Mutat Res, 2005. 584(1-2): p. 1-256.
4. Chhabra, R.S., Environ Health Perspect, 1979. 33: p. 61-9.
5. Le Fevre, A.C., et al., Mutat Res, 2007. 619(1-2): p. 16-29.
6. Eun, J.W., et al., Toxicology, 2008. 249(2-3): p. 176-83.
7. Aubrecht, J., et al., Toxicol Appl Pharmacol, 1999. 154(3): p. 228-35.
8. Ellinger-Ziegelbauer, H., et al., Toxicol Lett, 2009. 186(1): p. 36-44.
9. Ellinger-Ziegelbauer, H., et al., Mutat Res, 2007.
10. Ellinger-Ziegelbauer, H., et al., Toxicol Sci, 2004. 77(1): p. 19-34.
11. Ellinger-Ziegelbauer, H., et al., Mutat Res, 2005. 575(1-2): p. 61-84.
12. Nioi, P., et al., Chem Biol Interact, 2008. 172(3): p. 206-15.
13. Waterston, R.H., et al., Nature, 2002. 420(6915): p. 520-62.
14. Koike, M., et al., J Radiat Res (Tokyo), 2008.
15. Rogakou, E.P., et al., J Biol Chem, 1998. 273(10): p. 5858-68.
16. Seglen, P.O., Methods Cell Biol, 1976. 13: p. 29-83.
17. Casciano, D.A., Drug Metab Rev, 2000. 32(1): p. 1-13.
18. Mathijs, K., et al., Drug Metab Dispos, 2009.
19. Koebe, H.G., et al., Int J Artif Organs, 1994. 17(2): p. 95-106.
20. Beken, S., et al., Methods Mol Biol, 1998. 107: p. 303-9.
21. Hamer, G., et al., Biol Reprod, 2003. 68(2): p. 628-34.
22. Tong, W., et al., Environ Health Perspect, 2003. 111(15): p. 1819-26.
23. Tong, W., et al., Mutat Res, 2004. 549(1-2): p. 241-53.
24. Irizarry, R.A., et al., Biostatistics, 2003. 4(2): p. 249-64.
25. Affymetrix, Statistical Algorithms Description Document, technical report. 2002.
26. Shi, L., et al., Nat Biotechnol, 2006. 24(9): p. 1151-61.
27. Tibshirani, R., et al., Proc Natl Acad Sci U S A, 2002. 99(10): p. 6567-72.
28. Fernandez-Capetillo, O., et al., DNA Repair (Amst), 2004. 3(8-9): p. 959-67.
29. Rogakou, E.P., et al., J Cell Biol, 1999. 146(5): p. 905-16.
30. Mladenov, E., I. Tsaneva, and B. Anachkova, J Cell Physiol, 2007. 211(2): p. 468-76.
31. Yamamoto, K., et al., Mol Med, 2008. 14(3-4): p. 167-74.
32. Zhou, C., et al., Mutat Res, 2006. 604(1-2): p. 8-18.
33. Hoogervorst, E.M., et al., DNA Repair (Amst), 2005. 4(1): p. 3-9.
34. Schrenk, D., et al., Carcinogenesis, 1994. 15(11): p. 2541-6.
35. Jenkins, G.J. and J.M. Parry, Teratog Carcinog Mutagen, 2000. 20(3): p. 107-17.
36. Moller, M.E., et al., Carcinogenesis, 1984. 5(6): p. 797-804.
37. Vu, V.T., et al., Carcinogenesis, 1985. 6(1): p. 45-52.
38. Heflich, R.H. and R.E. Neft, Mutat Res, 1994. 318(2): p. 73-114.
39. Sionov, R.V. and Y. Haupt, Oncogene, 1999. 18(45): p. 6145-57.
40. lida, M., et al., Carcinogenesis, 2005. 26(3): p. 689-99.
41. Uehara, T., et al., Toxicology, 2008. 250(1): p. 15-26.
42. van Delft, J.H., et al., Carcinogenesis, 2004. 25(7): p. 1265-76.

## Claims

1. In vitro method for distinguishing between genotoxic and non-genotoxic compounds by determining the expression level of at least genes 1700007K13Rik, GAS2L3, SPC25 and DDIT4L in primary mouse hepatocytes exposed to a potentially genotoxic compound and comparing the expression level thus obtained with a normal value of expression of said genes wherein it is concluded that a compound is genotoxic if the expression of at least one of said genes is increased at least two-fold.

2. Method according to claim 1 wherein the primary hepatocytes are exposed to the potentially genotoxic compound at two different points in time.

3. Method according to claim 2 wherein the two points in time are about 24 hours and about 48 hours.

4. Method according to claims 1 - 3 wherein the expression values of genes 1700007K13Rik GAS2L3, SPC25 and DDIT4L are measured in two or more independent samples.

5. Method according to claims 1 - 4 wherein it is concluded that a compound is genotoxic if the expression of at least two of said genes are increased at least two-fold.

6. Method according to claim 5 wherein it is concluded that a compound is genotoxic if the expression of at least three of said genes are increased at least two-fold.

7. Method according to claim 6 wherein it is concluded that a compound is genotoxic if the expression of all 4 genes are increased at least two-fold.

## Patentansprüche

1. In-vitro-Verfahren zum Unterscheiden zwischen genotoxischen und nichtgenotoxischen Verbindungen durch Bestimmen des Expressionsniveaus wenigstens der Gene 1700007K13Rik, GAS2L3, SPC25 und DDIT4L in primären Maus-Hepatozyten, exponiert gegenüber einer potenziell genotoxischen Verbindung, und Vergleichen des so erhaltenen Expressionsniveaus mit einem Normalwert der Expression der Gene, wobei geschlossen wird, dass eine Verbindung genotoxisch ist, wenn die Expression wenigstens eines der Gene wenigstens zweifach erhöht ist.

2. Verfahren gemäß Anspruch 1, wobei die primären Hepatozyten zu zwei verschiedenen Zeitpunkten gegenüber der potenziell genotoxischen Verbindung exponiert werden.

3. Verfahren gemäß Anspruch 2, wobei die beiden Zeitpunkte etwa 24 Stunden und etwa 48 Stunden betragen.

4. Verfahren gemäß Ansprüchen 1-3, wobei die Expressionswerte der Gene 1700007K13Rik, GAS2L3, SPC25 und DDIT4L in zwei oder mehr unabhängigen Proben gemessen werden.

5. Verfahren gemäß Ansprüchen 1-4, wobei geschlossen wird, dass eine Verbindung genotoxisch ist, wenn die Expression von wenigstens zwei der Gene wenigstens zweifach erhöht ist.

6. Verfahren gemäß Anspruch 5, wobei geschlossen wird, dass eine Verbindung genotoxisch ist, wenn die Expression von wenigstens drei der Gene wenigstens zweifach erhöht ist.

7. Verfahren gemäß Anspruch 6, wobei geschlossen wird, dass eine Verbindung genotoxisch ist, wenn die Expression aller 4 Gene wenigstens zweifach erhöht ist.

## Revendications

1. Procédé in vitro pour distinguer entre des composés génotoxiques et non génotoxiques par détermination du niveau d'expression d'au moins les gènes 1700007K13Rik, GAS2L3, SPC25 et DDIT4L dans des hépatocytes de souris primaires exposés à un composé potentiellement génotoxique et comparaison du niveau d'expression ainsi obtenu à une valeur d'expression normale desdits gènes où il est conclu qu'un composé est génotoxique si l'expression d'au moins un desdits gènes est augmentée au moins deux fois.

2. Procédé selon la revendication 1 dans lequel les hépatocytes primaires sont exposés au composé potentiellement génotoxique à deux temps différents.

3. Procédé selon la revendication 2 dans lequel les deux temps sont d'environ 24 heures et d'environ 48 heures.

4. Procédé selon les revendications 1 à 3 dans lequel les valeurs d'expression des gènes 1700007K13Rik, GAS2L3, SPC25 et DDIT4L sont mesurées dans deux échantillons indépendants ou plus.

5. Procédé selon les revendications 1 à 4 dans lequel il est conclu qu'un composé est génotoxique si l'expression d'au moins deux desdits gènes est augmentée au moins deux fois.

6. Procédé selon la revendication 5 dans lequel il est conclu qu'un composé est génotoxique si l'expression d'au moins trois desdits gènes est augmentée au moins deux fois.

7. Procédé selon la revendication 6 dans lequel il est conclu qu'un composé est génotoxique si l'expression des 4 gènes est augmentée au moins deux fois.
